# EUROPEAN PATENT APPLICATION

(11) **EP 4 792 748 A1**
(43) Date of publication of application: **19.08.2026**
(21) Application number: 24877039.8
(22) Date of filing: 27.09.2024
(51) Int. Cl.: A61B 6/42

(54) **X-RAY IMAGING DEVICE**

(30) Priority: 11.10.2023 JP 2023176134
(71) Applicant: SHIMADZU CORPORATION, Kyoto-shi, Kyoto 604-8511 (JP)
(72) Inventor: MIWA, Takatoshi, Kyoto-shi, Kyoto 604-8511 (JP); KOJIMA, Miharu, Kyoto-shi, Kyoto 604-8511 (JP); SHIMAZU, Keisuke, Kyoto-shi, Kyoto 604-8511 (JP)
(74) Representative: Müller-Boré & Partner Patentanwälte PartG mbB
(86) International application number: PCT/JP2024/034636
(87) International publication number: WO 2025/079440

(57) **Abstract**

This X-ray imaging apparatus (100, 110) includes a column (17), an X-ray detection unit (20), a support unit (21) that is provided on the column and supports the X-ray detection unit so as to be movable in a vertical direction, a grip unit (23) that is provided so as to move in the vertical direction integrally with the X-ray detection unit in accordance with movement of the X-ray detection unit in the vertical direction, and a first moving mechanism (33) that is provided on the support unit and relatively moves the grip unit in the vertical direction with respect to the X-ray detection unit.

## Description

### Technical Field

The present invention relates to an X-ray imaging apparatus.

### Background Art

Conventionally, an X-ray imaging apparatus including an X-ray detection unit and a grip unit is known. Such an X-ray imaging apparatus is disclosed in, for example, Japanese Unexamined Patent Application Publication No. 2016-67842.

The above-mentioned Japanese Unexamined Patent Application Publication No. 2016-67842 discloses an X-ray imaging apparatus including an apparatus main body (X-ray detection unit) and a grip bar (grip unit). The apparatus main body is moved in a vertical direction by an image receiving unit drive unit. The image receiving unit drive unit is provided in a column. The grip bar is provided to be gripped by a subject when imaging the subject from a side surface. The grip bar is moved in the vertical direction by a grip bar drive unit. The grip bar drive unit is provided in a casing attached to a back surface of the column.

### Prior Art Documents

### Patent Literature

Patent Document 1: Japanese Unexamined Patent Application Publication No. 2016-67842

### Summary of Invention

### Technical Problem

However, in the X-ray imaging apparatus as disclosed in the above-mentioned Japanese Unexamined Patent Application Publication No. 2016-67842, in order to cope with imaging of an imaging region, such as a chest or lower limbs, the position of which varies depending on the subject, for subjects with different heights, the apparatus main body (X-ray detection unit) is configured to be movable over a long distance in the vertical direction along the column. Furthermore, the grip bar (grip unit) is moved in the vertical direction by the grip bar drive unit so as to correspond to the movement of the apparatus main body over a long distance in the vertical direction. Therefore, the grip bar drive unit has a configuration in which a stroke length in the vertical direction is large, resulting in a large-scale configuration of the grip bar drive unit. Therefore, it is desired to suppress an increase in size of a moving mechanism that moves the grip unit in the vertical direction.

The present invention has been made to solve the above-described problems, and one object of the present invention is to provide an X-ray imaging apparatus capable of suppressing an increase in size of a moving mechanism that moves a grip unit in a vertical direction.

### Solution to Problem

An X-ray imaging apparatus according to a first aspect of the present invention includes: an X-ray irradiation unit that irradiates X-rays; a column; an X-ray detection unit that detects the X-rays irradiated from the X-ray irradiation unit and is movable in a vertical direction along the column; a support unit that is provided on the column and supports the X-ray detection unit so as to be movable in the vertical direction; a grip unit that is configured to be gripped by a hand of a subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.

An X-ray imaging apparatus according to a second aspect of the present invention includes: a column; an X-ray detection unit that detects X-rays transmitted through a subject and is movable in a vertical direction along the column; a support unit that is provided on the column and supports the X-ray detection unit so as to be movable in the vertical direction; a grip unit that is configured to be gripped by a hand of the subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.

### Advantageous Effects of Invention

In the X-ray imaging apparatus according to the first aspect and the second aspect, there are provided: the support unit that is provided on the column and supports the X-ray detection unit so as to be movable in the vertical direction; the grip unit that is provided so as to move in the vertical direction integrally with the X-ray detection unit in accordance with the movement of the X-ray detection unit in the vertical direction; and the first moving mechanism that is provided on the support unit and relatively moves the grip unit in the vertical direction with respect to the X-ray detection unit. Here, for example, when chest imaging is performed from the side surface of the subject, the grip unit is gripped by the hand of the subject in a state where the hand of the subject is raised, whereby it is possible to suppress the arm of the subject from being reflected in the image. Therefore, the position of the grip unit in the vertical direction may be any position as long as it can be gripped by the hand of the subject in a state where the hand of the subject is raised. For this reason, clinically, for example, in chest imaging from the side surface of the subject, the X-ray detection unit and the grip unit are not excessively separated from each other. Further, although the position of the X-ray detection unit in the vertical direction at the time of imaging differs between a tall subject and a short subject, it is not necessary to increase the stroke length of the grip unit in the vertical direction if the position of the grip unit is adjusted based on the position of the X-ray detection unit in the vertical direction. Focusing on these points, in the present invention, the grip unit is provided so as to move in the vertical direction integrally with the X-ray detection unit in accordance with the movement of the X-ray detection unit in the vertical direction, and the first moving mechanism is provided on the support unit and is configured to relatively move the grip unit in the vertical direction with respect to the X-ray detection unit. As a result, since both the X-ray detection unit and the first moving mechanism that relatively moves the grip unit in the vertical direction are provided on the support unit, the integral movement of the X-ray detection unit and the grip unit in accordance with the movement of the X-ray detection unit in the vertical direction can be easily performed. Therefore, since the stroke length of the first moving mechanism can be reduced, it is possible to suppress an increase in size of the first moving mechanism that moves the grip unit in the vertical direction.

### Brief Description of Drawings

[FIG. 1] FIG. 1 is a schematic diagram showing an outline of an X-ray imaging apparatus according to a first embodiment.
[FIG. 2] FIG. 2 is a schematic diagram showing an apparatus configuration of the X-ray imaging apparatus according to the first embodiment.
[FIG. 3] FIG. 3 is a perspective view of the X-ray imaging apparatus according to the first embodiment.
[FIG. 4] FIG. 4 is a schematic diagram for explaining relative movement of a grip unit in a vertical direction by a first moving mechanism.
[FIG. 5] FIG. 5 is a block diagram showing a control configuration of the X-ray imaging apparatus according to the first embodiment.
[FIG. 6] FIG. 6 is a first schematic diagram for explaining adjustment of a position of an X-ray detection unit according to the first embodiment.
[FIG. 7] FIG. 7 is a second schematic diagram for explaining adjustment of the position of the X-ray detection unit according to the first embodiment. [FIG. 8] FIG. 8 is a block diagram showing a control configuration of an X-ray imaging apparatus according to a second embodiment.
[FIG. 9] FIG. 9 is a schematic diagram showing an apparatus configuration of the X-ray imaging apparatus according to the second embodiment.
[FIG. 10] FIG. 10 is a schematic diagram showing a second moving mechanism according to the second embodiment.
[FIG. 11] FIG. 11 is a first schematic diagram for explaining adjustment of a position of an X-ray detection unit according to the second embodiment.
[FIG. 12] FIG. 12 is a second schematic diagram for explaining adjustment of the position of the X-ray detection unit according to the second embodiment.

### Description of Embodiments

Hereinafter, embodiments embodying the present invention will be described with reference to the drawings.

### [First Embodiment]

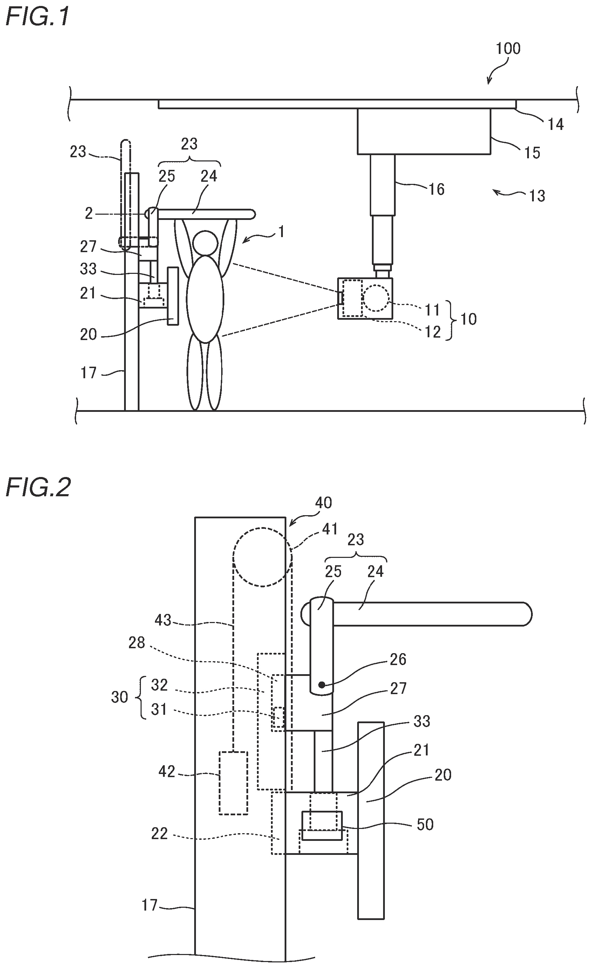

An outline of an X-ray imaging apparatus 100 according to a first embodiment will be described with reference to FIGS. 1 to 7.

### (Outline of X-ray Imaging Apparatus)

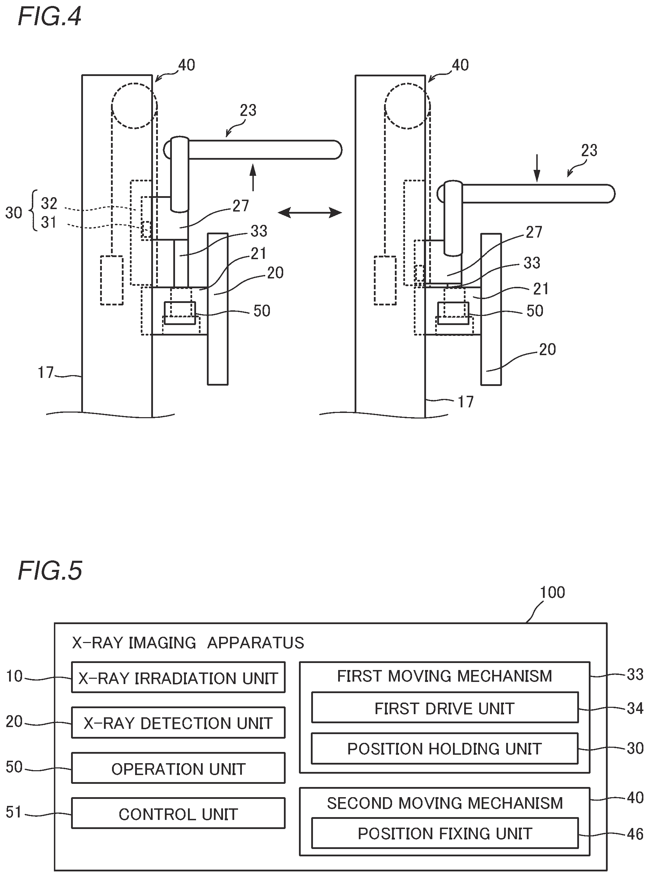

As shown in FIG. 1, the X-ray imaging apparatus 100 includes an X-ray irradiation unit 10, an irradiation unit holding mechanism 13, a column 17, an X-ray detection unit 20, a support unit 21, a grip unit 23, a connection member 27, a position holding unit 30 (see FIG. 2), a first moving mechanism 33, a second moving mechanism 40 (see FIG. 2), an operation unit 50 (see FIG. 2), and a control unit 51 (see FIG. 5).

The X-ray irradiation unit 10 includes an X-ray tube 11 and a collimator 12. The X-ray tube 11 is configured to irradiate a subject 1 with X-rays. The collimator 12 is configured to adjust an irradiation field of the X-rays irradiated from the X-ray tube 11.

The irradiation unit holding mechanism 13 is configured to movably hold the X-ray irradiation unit 10. The irradiation unit holding mechanism 13 includes a ceiling suspension device 15 and a column member 16. The irradiation unit holding mechanism 13 is supported by rails 14 provided on a ceiling of an imaging room. The ceiling suspension device 15 is configured to be movable in a horizontal direction by the rails 14. The ceiling suspension device 15 is configured to support the column member 16. The column member 16 is configured to support the X-ray irradiation unit 10. The column member 16 is configured to be extendable and contractible in a vertical direction. The X-ray irradiation unit 10 is configured to be movable in the vertical direction by the column member 16.

The column 17 is configured to support the X-ray detection unit 20. The column 17 is configured to support the X-ray detection unit 20 via the support unit 21 so as to be movable in the vertical direction. The column 17 is an imaging stand for performing X-ray imaging in a posture (standing position) in which the subject 1 is standing. During X-ray imaging, the X-ray irradiation unit 10 is disposed at a position horizontally facing the X-ray detection unit 20 provided on the column 17 via the support unit 21. The X-ray imaging apparatus 100 images the subject 1 standing in front of the column 17 between the X-ray tube 11 and the X-ray detection unit 20 that face each other horizontally.

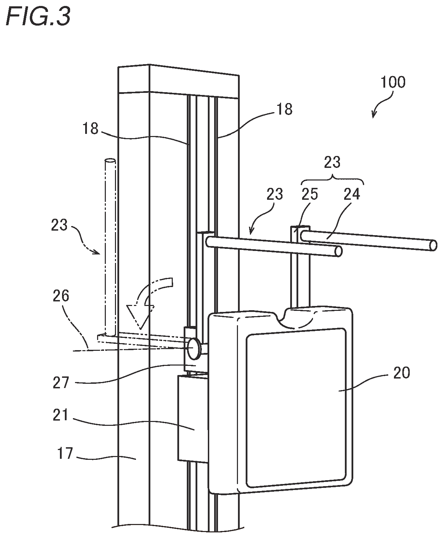

The column 17 includes guide units 18 (see FIG. 3) provided so as to extend in the vertical direction. The guide units 18 are provided on a left side and a right side of the column 17, respectively. The guide unit 18 is formed so as to guide the support unit 21 when the support unit 21 moves up and down together with the X-ray detection unit 20 and the first moving mechanism 33. That is, the support unit 21 moves up and down while being guided by the guide unit 18 together with the X-ray detection unit 20 and the first moving mechanism 33. Further, the guide unit 18 is formed so as to guide the connection member 27 when the connection member 27 moves up and down together with the grip unit 23. That is, the connection member 27 moves up and down while being guided by the guide unit 18 together with the grip unit 23. The guide unit 18 has a groove shape that engages with the support unit 21 and the connection member 27. Note that the structure of the guide unit 18 is not particularly limited as long as it can engage with and guide the support unit 21 and also engage with and guide the connection member 27.

The X-ray detection unit 20 is configured to detect X-rays irradiated from the X-ray irradiation unit 10 and transmitted through the subject 1. The X-ray detection unit 20 includes, for example, an FPD (Flat Panel Detector). The X-ray detection unit 20 is provided on the column 17 via the support unit 21. The X-ray detection unit 20 is configured to be movable in the vertical direction along the column 17 via the support unit 21.

As shown in FIG. 2, the support unit 21 is provided on the column 17 and is configured to support the X-ray detection unit 20 so as to be movable in the vertical direction. The support unit 21 is configured such that the X-ray detection unit 20 is movable in the vertical direction by the second moving mechanism 40. The support unit 21 is disposed between the column 17 and the X-ray detection unit 20. The X-ray detection unit 20 is attached to the support unit 21 on a side opposite to the column 17.

The support unit 21 includes a first guided unit 22 provided on the column 17 side. The first guided unit 22 is guided by the guide unit 18 (see FIG. 3) formed on the column 17. The first guided unit 22 has a convex shape protruding toward the guide unit 18 side. Two first guided units 22 each having a convex shape are provided so as to extend along the vertical direction and to face each other on the left and right. The convex-shaped first guided unit 22 engages with the groove-shaped guide unit 18. As a result, the first guided unit 22 is guided by the guide unit 18.

As shown in FIG. 1, the grip unit 23 is configured to be gripped by the hand of the subject 1, for example, when chest imaging is performed from the side surface of the subject 1. The grip unit 23 is provided on the connection member 27. One grip unit 23 (see FIG. 3) is provided on each of the left side and the right side of the connection member 27. Further, the connection member 27 is provided at an upper portion of the first moving mechanism 33. Further, the first moving mechanism 33 is provided on the support unit 21. Therefore, the grip unit 23 moves in the vertical direction integrally with the X-ray detection unit 20 via the connection member 27 in accordance with the movement of the X-ray detection unit 20 in the vertical direction. Note that the grip unit 23 may be configured to support the hand or arm of the subject 1 without being gripped by the hand of the subject 1.

As shown in FIG. 3, the grip unit 23 includes a first member 24 and a second member 25. The first member 24 is gripped by the hand of the subject 1. The first member 24 has a bar shape. The second member 25 connects the first member 24 and the connection member 27. The second member 25 has a bar shape. The second member 25 is connected to the first member 24 so as to intersect therewith, and is rotatably attached to the connection member 27. The grip unit 23 is configured to rotate around a rotation axis 26 (see FIG. 2). Specifically, the grip unit 23 is attached to the connection member 27 so as to be rotatable between a grippable position where the first member 24 extends substantially horizontally toward the X-ray irradiation unit 10 and can be gripped by the hand of the subject 1, and a retracted position where the first member 24 extends substantially vertically along the column 17 and suppresses contact between the first member 24 and the subject 1. The first member 24 of the grip unit 23 is disposed above an upper end of the X-ray detection unit 20 at the grippable position. Note that, in FIG. 3, the grip unit 23 at the retracted position is represented by a two-dot chain line.

Here, since the grip unit 23 is configured to move in the vertical direction integrally with the X-ray detection unit 20 in accordance with the movement of the X-ray detection unit 20 in the vertical direction, the stroke length of the first moving mechanism 33 can be reduced as compared with a case where the grip unit 23 is not provided so as to move integrally with the X-ray detection unit 20. As a result, there is a limit to the upper position where the grip unit 23 can be retracted by the first moving mechanism 33. Therefore, the grip unit 23 is configured to be able to suppress contact between the first member 24 and the subject 1 by rotating from the grippable position to the retracted position.

As shown in FIG. 2, the connection member 27 is configured to connect the second member 25 of the grip unit 23 and the first moving mechanism 33. An upper portion of the first moving mechanism 33 is attached to a lower portion of the connection member 27. The second member 25 of the grip unit 23 is rotatably attached to an upper portion of the connection member 27. Further, an electromagnet 31 included in the position holding unit 30 is provided on the connection member 27.

The connection member 27 includes a second guided unit 28 provided on the column 17 side. The second guided unit 28 is guided by the guide unit 18 (see FIG. 3) formed on the column 17. The second guided unit 28 has a convex shape protruding toward the guide unit 18 side. Two second guided units 28 each having a convex shape are provided so as to extend along the vertical direction and to face each other on the left and right. The convex-shaped second guided unit 28 engages with the groove-shaped guide unit 18. As a result, the second guided unit 28 is guided by the guide unit 18.

The position holding unit 30 is configured to hold the position of the grip unit 23 in the vertical direction with respect to the column 17. The position holding unit 30 holds the grip unit 23 at a predetermined position 2, for example, by magnetic force. The position holding unit 30 includes an electromagnet 31 and a magnetic body 32. The electromagnet 31 of the position holding unit 30 is provided on the connection member 27. The magnetic body 32 of the position holding unit 30 is, for example, a metal plate such as an iron plate. The magnetic body 32 of the position holding unit 30 is provided on the column 17. When the electromagnet 31 of the position holding unit 30 is energized at the predetermined position 2, the grip unit 23 is held at the predetermined position 2 by a magnetic force between the electromagnet 31 of the position holding unit 30 and the magnetic body 32 included in the position holding unit 30 provided on the column 17. Here, the "predetermined position" in the present specification means a position of the grip unit 23 such that the arm of the subject 1 is not reflected in the X-ray imaging in a state where the hand of the subject 1 is raised and the grip unit 23 is gripped by the hand of the subject 1.

As shown in FIG. 4, the first moving mechanism 33 is configured to relatively move the grip unit 23 in the vertical direction with respect to the X-ray detection unit 20. Specifically, the first moving mechanism 33 relatively moves the grip unit 23 in the vertical direction with respect to the X-ray detection unit 20 by moving the connection member 27 in the vertical direction. The first moving mechanism 33 is, for example, a linear motion actuator (linear actuator), and includes a first drive unit 34 (see FIG. 5) such as a motor. The first moving mechanism 33 is provided on the support unit 21 disposed between the column 17 and the X-ray detection unit 20. The upper portion of the first moving mechanism 33 is connected to the connection member 27. Based on an operation input from the operation unit 50, the grip unit 23 is relatively moved in the vertical direction with respect to the X-ray detection unit 20 via the connection member 27 by the first drive unit 34. Driving of the first drive unit 34 is controlled by the control unit 51. Note that the configuration of the first moving mechanism 33 is not particularly limited, and known mechanisms and members can be used.

As shown in FIG. 2, the second moving mechanism 40 is configured to move the X-ray detection unit 20 in the vertical direction via the support unit 21. Specifically, the second moving mechanism 40 moves the X-ray detection unit 20 in the vertical direction by moving the support unit 21 in the vertical direction. The second moving mechanism 40 is provided on the column 17. As an example, the second moving mechanism 40 includes a pulley 41, a counterweight 42, a belt 43, and a position fixing unit 46 (see FIG. 5). In the first embodiment, the X-ray detection unit 20 is moved in the vertical direction by the second moving mechanism 40 manually by a radiological technologist.

Furthermore, based on an operation input from the operation unit 50, the position of the X-ray detection unit 20 in the vertical direction is fixed by the position fixing unit 46 (see FIG. 5). The position fixing unit 46 includes an electromagnet. Based on an operation input from the operation unit 50, the control unit 51 restricts rotation of the pulley 41 by a magnetic force of the electromagnet.

The operation unit 50 is configured to receive an operation input for operating the X-ray imaging apparatus 100. As an example, the operation unit 50 receives an operation input for holding the grip unit 23 by the position holding unit 30, relatively moving the grip unit 23 in the vertical direction by the first drive unit 34 (see FIG. 5), and the like. The operation unit 50 includes a plurality of operation buttons. The above-described various operations are inputted to the control unit 51 by the radiological technologist operating the operation buttons. As an example, when an operation for relatively moving the grip unit 23 upward by the first drive unit 34 is received by the operation unit 50, the control unit 51 performs control to relatively move the grip unit 23 upward by the first drive unit 34.

The operation unit 50 is provided on a side surface of the support unit 21. The position of the operation unit 50 is not particularly limited, and for example, it may be provided on a side surface of the X-ray detection unit 20 or may be provided on the column 17. Further, the operation unit 50 may be a remote controller that receives the above-described various operation inputs, or may be a terminal such as a smartphone or a tablet in which application software for the above-described various operations is installed.

The control unit 51 (see FIG. 5) includes a processor such as a CPU (Central Processing Unit). The control unit 51 (processor) functions as a control unit 51 that controls the position holding unit 30 and the first drive unit 34 by executing an operation program stored in a storage unit (not shown).

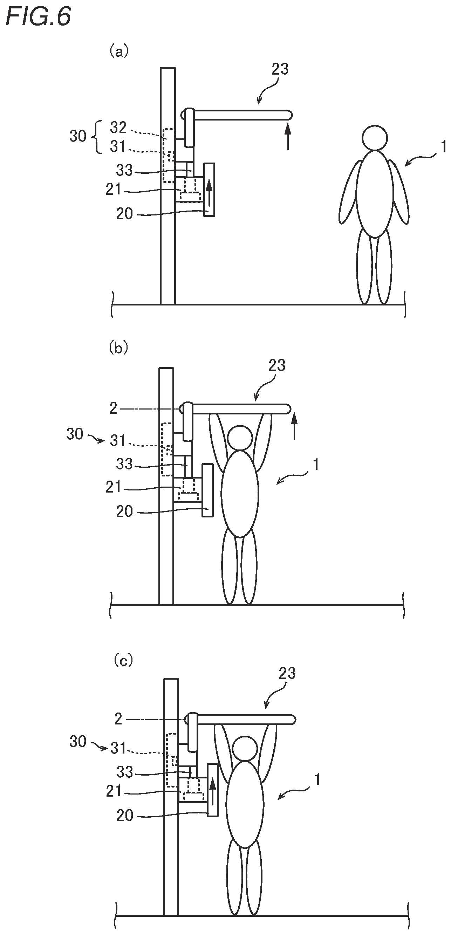

After the position of the grip unit 23 is positioned at the predetermined position 2 with respect to the column 17, the control unit 51 performs control to move the X-ray detection unit 20 in the vertical direction by the first drive unit 34 (see FIG. 5) while holding the position of the grip unit 23. Specifically, as shown in FIG. 6(c), in the adjustment of the position of the X-ray detection unit 20, after the positioning of the grip unit 23, the control unit 51 performs control so that the position holding unit 30 holds the position of the grip unit 23 with respect to the column 17, and the first drive unit 34 relatively moves the X-ray detection unit 20 in the vertical direction with respect to the grip unit 23. Thus, the position of the X-ray detection unit 20 is adjusted.

(Adjustment of Position of X-ray Detection Unit by First Drive Unit) Adjustment of the position of the X-ray detection unit 20 in the vertical direction by the first drive unit 34 (see FIG. 5) when chest imaging is performed from the side surface of the subject 1 will be described with reference to FIGS. 6(a) to 6(c).

As shown in FIG. 6(a), after the subject 1 enters the imaging room, rough positioning of the X-ray detection unit 20 with respect to the chest of the subject 1 is performed manually by the radiological technologist so that the X-ray detection unit 20 is positioned at the chest of the subject 1. That is, the X-ray detection unit 20 is manually moved in the vertical direction via the second moving mechanism 40 (see FIG. 2) by the radiological technologist. At this time, the grip unit 23 is also moved in the vertical direction integrally with the X-ray detection unit 20 via the connection member 27 in accordance with the movement of the X-ray detection unit 20 in the vertical direction. As an example, in FIG. 6(a), the X-ray detection unit 20 is moved upward via the second moving mechanism 40 manually by the radiological technologist.

Then, the grip unit 23 is moved in the vertical direction by an operation input to the operation unit 50 by the radiological technologist so as to be at a position where the grip unit 23 can be gripped by the hand of the subject 1 in a state where the hand of the subject 1 is raised. Then, as shown in FIG. 6(b), in a state where the hand of the subject 1 is raised and the grip unit 23 is gripped by the hand of the subject 1, the position of the grip unit 23 in the vertical direction is adjusted by an operation input to the operation unit 50 by the radiological technologist so that the arm of the subject 1 is not reflected in chest imaging. That is, according to the operation input to the operation unit 50 by the radiological technologist, the control unit 51 causes the first drive unit 34 to relatively move the grip unit 23 in the vertical direction with respect to the X-ray detection unit 20, and positions the position of the grip unit 23 at the predetermined position 2 with respect to the column 17. As an example, in FIG. 6(b), the control unit 51 relatively moves the grip unit 23 upward with respect to the X-ray detection unit 20 by the first drive unit 34.

Then, according to the operation input to the operation unit 50 by the radiological technologist, the control unit 51 energizes the electromagnet 31 of the position holding unit 30, and holds the grip unit 23 at the positioned position by the magnetic force between the electromagnet 31 and the magnetic body 32 of the position holding unit 30.

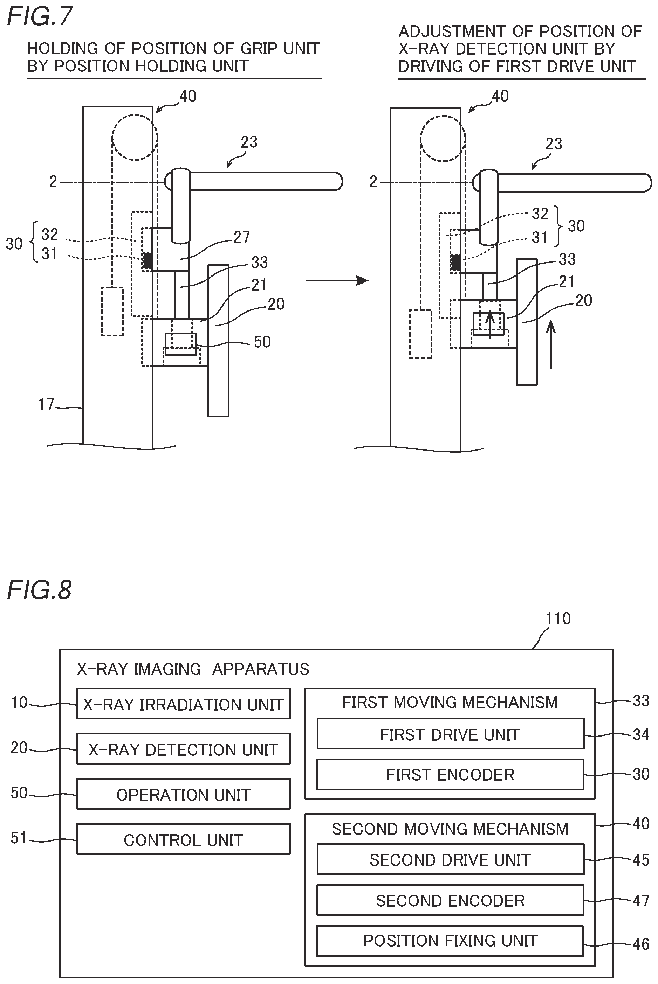

Then, as shown in FIGS. 6(c) and 7, in a state where the position of the grip unit 23 is held at the positioned position, the position of the X-ray detection unit 20 in the vertical direction with respect to the chest of the subject 1 is adjusted by the operation input to the operation unit 50 by the radiological technologist. Here, in a state where the position of the grip unit 23 with respect to the column 17 is held by the position holding unit 30, by driving the first drive unit 34, not the grip unit 23 but the X-ray detection unit 20 relatively moves in the vertical direction with respect to the grip unit 23 held at the positioned position. That is, according to the operation input to the operation unit 50 by the radiological technologist, the control unit 51 causes the first drive unit 34 to relatively move the X-ray detection unit 20 in the vertical direction with respect to the grip unit 23. Thus, the adjustment of the position of the X-ray detection unit 20 with respect to the chest of the subject 1 is performed by the first drive unit 34. As an example, in FIGS. 6(c) and 7, the control unit 51 relatively moves the X-ray detection unit 20 upward with respect to the grip unit 23 by the first drive unit 34.

Note that, after the adjustment of the position of the X-ray detection unit 20 with respect to the chest of the subject 1 by the first drive unit 34 is completed, the position of the grip unit 23 in the vertical direction may be finely adjusted. Specifically, after completion of the adjustment of the position of the X-ray detection unit 20 by the first drive unit 34, in accordance with the operation input to the operation unit 50 by the radiological technologist, the control unit 51 may stop the energization to the electromagnet 31 of the position holding unit 30 and the grip unit 23 may be relatively moved in the vertical direction with respect to the X-ray detection unit 20 by the first drive unit 34.

### (Effects of First Embodiment)

In the first embodiment, the following effects can be obtained.

In the first embodiment, as described above, there are provided: the support unit 21 that is provided on the column 17 and supports the X-ray detection unit 20 so as to be movable in the vertical direction; the grip unit 23 that is provided so as to move in the vertical direction integrally with the X-ray detection unit 20 in accordance with the movement of the X-ray detection unit 20 in the vertical direction; and the first moving mechanism 33 that is provided on the support unit 21 and relatively moves the grip unit 23 in the vertical direction with respect to the X-ray detection unit 20. Here, for example, in chest imaging from the side surface of the subject 1, the X-ray detection unit 20 and the grip unit 23 are not excessively separated from each other. Further, if the position of the grip unit 23 is adjusted based on the position of the X-ray detection unit 20 in the vertical direction, it is not necessary to increase the stroke length of the grip unit 23 in the vertical direction. By adopting the above configuration, both the X-ray detection unit 20 and the first moving mechanism 33 that relatively moves the grip unit 23 in the vertical direction are provided on the support unit 21, so that the integral movement of the X-ray detection unit 20 and the grip unit 23 associated with the movement of the X-ray detection unit 20 in the vertical direction can be easily performed. Therefore, since the stroke length of the first moving mechanism 33 can be reduced, it is possible to suppress an increase in size of the first moving mechanism 33 that moves the grip unit 23 in the vertical direction.

Further, in the above embodiment, by configuring as follows, the following further effects are obtained.

That is, in the first embodiment, as described above, the first moving mechanism 33 is provided on the support unit 21 disposed between the column 17 and the X-ray detection unit 20. Thus, contact with the column 17 and the X-ray detection unit 20 by the first moving mechanism 33 in the upward direction can be reliably suppressed.

Further, in the first embodiment, as described above, the first moving mechanism 33 includes the first drive unit 34, and further includes the operation unit 50 that receives an operation input for relatively moving the grip unit 23 in the vertical direction with respect to the X-ray detection unit 20 by the first drive unit 34. As a result, relative movement of the grip unit 23 in the vertical direction by the first drive unit 34 can be easily performed by using the operation unit 50.

Further, in the first embodiment, as described above, the control unit 51 that controls the driving of the first drive unit 34 is further provided, and after the position of the grip unit 23 is positioned at the predetermined position 2 with respect to the column 17, the control unit 51 performs control to move the X-ray detection unit 20 in the vertical direction by the first drive unit 34 while holding the position of the grip unit 23, whereby the position of the X-ray detection unit 20 is adjusted. Thus, by driving the first drive unit 34, the position of the X-ray detection unit 20 after the position of the grip unit 23 is positioned at the predetermined position 2 with respect to the column 17 can be easily adjusted.

Further, in the first embodiment, as described above, the position holding unit 30 that holds the vertical position of the grip unit 23 with respect to the column 17 is further provided, and in the adjustment of the position of the X-ray detection unit 20, after positioning the grip unit 23, the control unit 51 performs control to hold the position of the grip unit 23 with respect to the column 17 by the position holding unit 30 and to relatively move the X-ray detection unit 20 in the vertical direction with respect to the grip unit 23 by the first drive unit 34. Thus, since the position of the grip unit 23 with respect to the column 17 is held by the position holding unit 30, not the grip unit 23 but the X-ray detection unit 20 can be relatively moved in the vertical direction with respect to the grip unit 23 by driving the first drive unit 34. Therefore, the adjustment of the position of the X-ray detection unit 20 after the position of the grip unit 23 is positioned at the predetermined position 2 with respect to the column 17 can be appropriately performed.

Further, in the first embodiment, as described above, the position holding unit 30 includes the electromagnet 31 and the magnetic body 32, and holds the grip unit 23 at the predetermined position 2 by a magnetic force between the electromagnet 31 and the magnetic body 32. As a result, the grip unit 23 can be easily and reliably held at the predetermined position 2 by switching the energization to the electromagnet 31.

Further, in the first embodiment, as described above, the column 17 includes the guide unit 18 provided so as to extend in the vertical direction, and the support unit 21 moves up and down while being guided by the guide unit 18 together with the first moving mechanism 33. As a result, since the support unit 21 moves up and down while being guided by the guide unit 18, it is possible to appropriately guide up-and-down movement of the support unit 21 and the first moving mechanism 33 provided on the support unit 21.

Further, in the first embodiment, as described above, the connection member 27 that connects the grip unit 23 and the first moving mechanism 33 is further provided, the connection member 27 moves up and down while being guided by the guide unit 18, and the support unit 21 moves up and down while being guided by the guide unit 18. This makes it possible to suppress horizontal swinging of both the X-ray detection unit 20 and the grip unit 23 when the X-ray detection unit 20 and the grip unit 23 move in the vertical direction.

### [Second Embodiment]

Next, a second embodiment of the present invention will be described with reference to FIGS. 8 to 11. In the second embodiment, unlike the first embodiment described above in which a drive unit for moving the X-ray detection unit 20 in the vertical direction is not provided, an example will be described in which a second moving mechanism 40 includes a second drive unit 45 and moves the X-ray detection unit 20 in the vertical direction. The same configurations as those in the first embodiment are denoted by the same reference numerals, and description thereof will be omitted.

As shown in FIG. 8, the second moving mechanism 40 includes a second drive unit 45 such as a motor. Driving of the second drive unit 45 is controlled by the control unit 51. In the second embodiment, the position holding unit 30 is not provided.

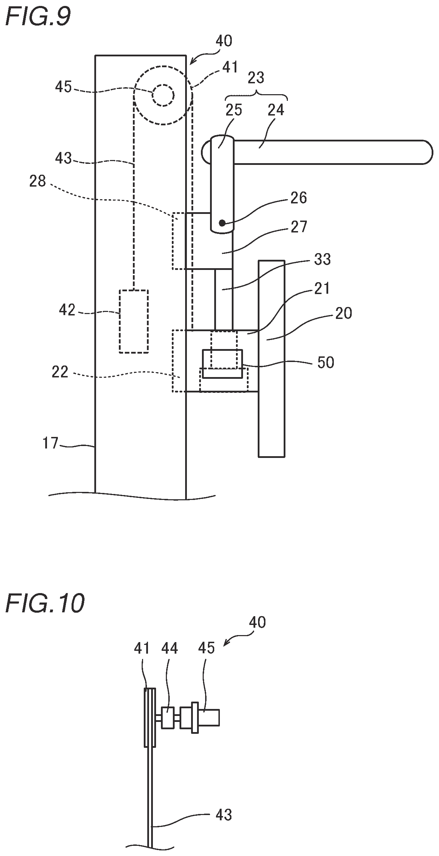

As shown in FIG. 9, the second moving mechanism 40 is configured to move the X-ray detection unit 20 in the vertical direction via the support unit 21. Specifically, the second moving mechanism 40 moves the X-ray detection unit 20 in the vertical direction by moving the support unit 21 in the vertical direction. The second moving mechanism 40 is provided on the column 17. As shown in FIG. 10, the second moving mechanism 40 includes, as an example, a pulley 41, a counterweight 42, a belt 43, a clutch 44, and a second drive unit 45.

The clutch 44 is an electromagnetic clutch. The second drive unit 45 is, for example, a motor or the like. The clutch 44 is configured to transmit and release transmission of power of the second drive unit 45 to the pulley 41. Driving of the second drive unit 45 and the clutch 44 is controlled by the control unit 51. When the clutch 44 is disengaged, the X-ray detection unit 20 is moved in the vertical direction by the second moving mechanism 40 manually by a radiological technologist. When the clutch 44 is engaged, the X-ray detection unit 20 is moved in the vertical direction by the second moving mechanism 40 in accordance with an operation input to the operation unit 50 by the radiological technologist. The belt 43 is wound around the pulley 41. A counterweight 42 is attached to one end of the belt 43. The support unit 21 is attached to the other end of the belt 43. Note that the configuration of the second moving mechanism 40 is not particularly limited, and known mechanisms and members can be used.

As shown in FIG. 8, the operation unit 50 is configured to receive an operation input for operating the X-ray imaging apparatus 110. As an example, the operation unit 50 receives an operation input for performing relative movement of the grip unit 23 in the vertical direction by the first drive unit 34, switching of the clutch 44, movement of the X-ray detection unit 20 in the vertical direction by the second drive unit 45, and the like. Further, the operation unit 50 receives an operation input for causing the first drive unit 34 and the second drive unit 45 to relatively move the X-ray detection unit 20 in the vertical direction with respect to the grip unit 23 while holding the position of the grip unit 23. The operation unit 50 includes a plurality of operation buttons. The above-described various operations are inputted to the control unit 51 by the radiological technologist operating the operation buttons.

After the position of the grip unit 23 is positioned at the predetermined position 2 with respect to the column 17, the control unit 51 performs control to move the X-ray detection unit 20 in the vertical direction by the first drive unit 34 while holding the position of the grip unit 23. Specifically, in the adjustment of the position of the X-ray detection unit 20, after the grip unit 23 is positioned, the control unit 51 performs control to relatively move the X-ray detection unit 20 in the vertical direction with respect to the grip unit 23 while holding the position of the grip unit 23 by moving the grip unit 23 and the X-ray detection unit 20 in mutually opposite directions at the same moving speed by the first drive unit 34 and the second drive unit 45 in a state where the clutch 44 is engaged. Thus, the position of the X-ray detection unit 20 is adjusted.

The first moving mechanism 33 includes a first encoder 35 attached to a motor serving as the first drive unit 34. Further, the second moving mechanism 40 includes a second encoder 47 attached to a motor serving as the first drive unit 34. When the control unit 51 performs control to relatively move the X-ray detection unit 20 in the vertical direction by the first drive unit 34 and the second drive unit 45, the control unit 51 is configured to acquire the moving speed of the X-ray detector from the second encoder 47 attached to the second drive unit 45, and to synchronize the moving speed of the grip unit 23 and the moving speed of the X-ray detection unit 20 by controlling the rotation of the first drive unit 34 based on the acquired moving speed of the X-ray detector. Further, when performing the control to relatively move the X-ray detection unit 20 in the vertical direction by the first drive unit 34 and the second drive unit 45, the control unit 51 is configured to move the grip unit 23 and the X-ray detection unit 20 in mutually opposite directions. That is, the control unit 51 performs control to relatively move the X-ray detection unit 20 in the vertical direction with respect to the grip unit 23 by the first drive unit 34 and the second drive unit 45 while holding the position of the grip unit 23.

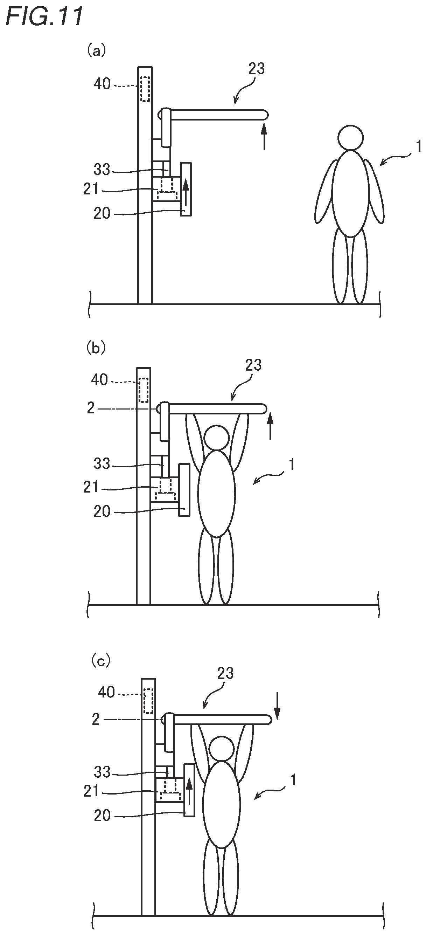

(Adjustment of Position of X-ray Detection Unit by First Drive Unit and Second Drive Unit) Adjustment of the position of the X-ray detection unit 20 in the vertical direction by the first drive unit 34 and the second drive unit 45 when chest imaging is performed from the side surface of the subject 1 will be described with reference to FIGS. 11(a) to 11(c).

Also in the second embodiment, similarly to the first embodiment, rough alignment of the X-ray detection unit 20 with respect to the chest of the subject 1 shown in FIG. 11(a) and positioning of the grip unit 23 to the predetermined position 2 with respect to the column 17 shown in FIG. 11(b) are performed. The rough positioning of the X-ray detection unit 20 and the positioning of the grip unit 23 to the predetermined position 2 are performed by the second drive unit 45 in a state where the clutch 44 is engaged by the second drive unit 45 according to an operation input of the operation unit 50 by the radiological technologist. Note that the rough alignment of the X-ray detection unit 20 may be manually performed by the radiological technologist.

Then, as shown in FIG. 11(c), in a state where the position of the grip unit 23 is positioned at the predetermined position 2, the position of the X-ray detection unit 20 in the vertical direction with respect to the chest of the subject 1 is adjusted by the radiological technologist in accordance with an operation input of the operation unit 50 in a state where the clutch 44 is engaged. An operation button (not shown) for relatively moving the X-ray detection unit 20 in the vertical direction by the first drive unit 34 and the second drive unit 45 is operated by the radiological technologist. Here, by moving the X-ray detection unit 20 by the second drive unit 45, the grip unit 23 also moves in the same direction integrally with the X-ray detection unit 20, but the position of the grip unit 23 can be held at the positioned predetermined position 2 by moving the grip unit 23 at the same moving speed in a direction opposite to the moving direction of the X-ray detection unit 20 by the first drive unit 34. Therefore, the control unit 51 relatively moves the X-ray detection unit 20 in the vertical direction with respect to the grip unit 23 by the first drive unit 34 and the second drive unit 45 while the position of the grip unit 23 is held at the predetermined position 2. As a result, the position of the X-ray detection unit 20 with respect to the chest of the subject 1 is adjusted by the first drive unit 34 and the second drive unit 45.

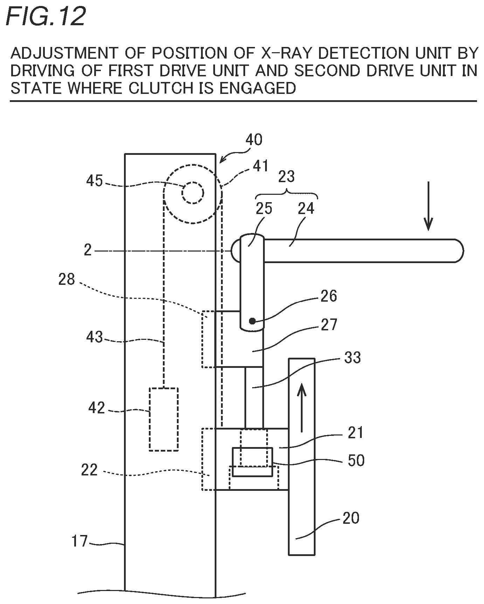

As an example, in FIGS. 11(c) and 12, the control unit 51 relatively moves the X-ray detection unit 20 upward with respect to the grip unit 23 by the first drive unit 34 and the second drive unit 45 while holding the position of the grip unit 23. By moving the X-ray detection unit 20 upward by the second drive unit 45, the grip unit 23 also moves upward integrally with the X-ray detection unit 20, but by moving the grip unit 23 downward, which is opposite to the moving direction of the X-ray detection unit 20, at the same moving speed by the first drive unit 34, the position of the grip unit 23 can be held at the positioned predetermined position 2. Therefore, the control unit 51 relatively moves the X-ray detection unit 20 upward with respect to the grip unit 23 by the first drive unit 34 and the second drive unit 45 while holding the position of the grip unit 23.

When the X-ray detection unit 20 is relatively moved downward with respect to the grip unit 23 by the first drive unit 34 and the second drive unit 45 while holding the position of the grip unit 23, contrary to FIGS. 11(c) and 12, the grip unit 23 also moves downward integrally with the X-ray detection unit 20 by moving the X-ray detection unit 20 downward by the second drive unit 45, but the position of the grip unit 23 can be held at the positioned predetermined position 2 by moving the grip unit 23 upward, which is opposite to the moving direction of the X-ray detection unit 20, at the same moving speed by the first drive unit 34. Therefore, the control unit 51 relatively moves the X-ray detection unit 20 downward with respect to the grip unit 23 by the first drive unit 34 and the second drive unit 45 while holding the position of the grip unit 23.

Other configurations of the second embodiment are the same as those of the first embodiment.

(Effects of Second Embodiment) In the second embodiment, the following effects can be obtained.

In the second embodiment, as described above, the second moving mechanism 40 including the second drive unit 45 and moving the X-ray detection unit 20 in the vertical direction is further provided, the control unit 51 is configured to control driving of the second drive unit 45, and in the adjustment of the position of the X-ray detection unit 20, after positioning the grip unit 23, the control unit 51 performs control to relatively move the X-ray detection unit 20 in the vertical direction with respect to the grip unit 23 while holding the position of the grip unit 23 by moving the grip unit 23 and the X-ray detection unit 20 in mutually opposite directions at the same moving speed by the first drive unit 34 and the second drive unit 45. Here, when the X-ray detection unit 20 is moved by the second drive unit 45, the grip unit 23 also moves in the same direction integrally with the X-ray detection unit 20, but the position of the grip unit 23 can be held by moving the grip unit 23 in a direction opposite to the moving direction of the X-ray detection unit 20 by the first drive unit 34. Therefore, the adjustment of the position of the X-ray detection unit 20 after the position of the grip unit 23 is positioned at the predetermined position 2 with respect to the column 17 can be appropriately performed.

Other effects of the second embodiment are the same as those of the first embodiment.

### [Modifications]

It should be considered that the embodiments disclosed herein are illustrative in all respects and not restrictive. The scope of the present invention is shown not by the description of the above embodiments but by the scope of the claims, and includes all changes (modifications) within the meaning and scope equivalent to the scope of the claims.

For example, in the first and second embodiments described above, the example in which one grip unit is provided on each of the left side and the right side of the connection member has been shown, but the present invention is not limited thereto. For example, the grip unit may be configured to be provided on either the left side or the right side of the connection member.

Further, in the first and second embodiments described above, the example in which the grip unit includes the first member and the second member has been shown, but the present invention is not limited thereto. For example, the grip unit may not include the second member, and the first member may be configured to be rotatably attached to the connection member.

Further, in the first and second embodiments described above, the example in which the first member of the grip unit has a bar shape has been shown, but the present invention is not limited thereto. For example, the first member of the grip unit may be configured to have an annular shape instead of a bar shape, or may be configured to have a curved shape.

Further, in the first and second embodiments described above, the example in which the grip unit and the first moving mechanism are connected via the connection member has been shown, but the present invention is not limited thereto. For example, the grip unit and the first moving mechanism may be configured to be directly connected without an intervening connection member.

Further, in the first embodiment described above, the example in which the position holding unit includes the electromagnet and the magnetic body has been shown, but the present invention is not limited thereto. For example, the position holding unit may be configured to include a member capable of clamping the column instead of the electromagnet and the magnetic body, or may be configured to include a member capable of engaging with and disengaging from the column.

Further, in the second embodiment described above, when the control unit performs control to relatively move the X-ray detection unit in the vertical direction by the first drive unit and the second drive unit, the control unit acquires the moving speed of the X-ray detector from the second encoder attached to the second drive unit, and synchronizes the moving speed of the grip unit and the moving speed of the X-ray detection unit by controlling the rotation of the first drive unit based on the acquired moving speed of the X-ray detector, but the present invention is not limited thereto. The method for synchronizing the moving speed is not particularly limited as long as the moving speed of the grip unit and the moving speed of the X-ray detection unit can be synchronized.

Further, in the second embodiment described above, an example in which the position holding unit is not provided has been shown, but the present invention is not limited thereto. For example, the position of the X-ray detection unit may be configured to be adjusted by any of control in which both the second drive unit and the position holding unit are provided, the position of the grip unit is held by the position holding unit, and the X-ray detection unit is relatively moved in the vertical direction with respect to the grip unit by the first drive unit, and control in which the X-ray detection unit is relatively moved in the vertical direction with respect to the grip unit by the first drive unit and the second drive unit while the position of the grip unit is held.

Further, in the first and second embodiments described above, an example in which the connection member includes the second guided unit has been shown, but the present invention is not limited thereto. For example, the connection member may be configured not to include the second guided unit.

[Aspects] It will be understood by those skilled in the art that the above-described exemplary embodiments are specific examples of the following aspects.

(Item 1) An X-ray imaging apparatus including: an X-ray irradiation unit that irradiates X-rays; a column; an X-ray detection unit that detects the X-rays irradiated from the X-ray irradiation unit and is movable in a vertical direction along the column; a support unit that is provided on the column and supports the X-ray detection unit so as to be movable in the vertical direction; a grip unit that is configured to be gripped by a hand of a subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.

(Item 2) The X-ray imaging apparatus according to item 1, wherein the first moving mechanism is provided on the support unit disposed between the column and the X-ray detection unit.

(Item 3) The X-ray imaging apparatus according to item 1 or 2, wherein the first moving mechanism includes a first drive unit, and further includes an operation unit that receives an operation input for relatively moving the grip unit in the vertical direction with respect to the X-ray detection unit by the first drive unit.

(Item 4) The X-ray imaging apparatus according to any one of items 1 to 3, wherein the first moving mechanism includes a first drive unit, further includes a control unit that controls driving of the first drive unit, and the control unit adjusts a position of the X-ray detection unit by performing control to move the X-ray detection unit in the vertical direction by the first drive unit while holding a position of the grip unit after the position of the grip unit is positioned at a predetermined position with respect to the column.

(Item 5) The X-ray imaging apparatus according to item 4, further including a position holding unit that holds a vertical position of the grip unit with respect to the column, wherein in the adjustment of the position of the X-ray detection unit, after positioning the grip unit, the control unit performs control to hold the position of the grip unit with respect to the column by the position holding unit and to relatively move the X-ray detection unit in the vertical direction with respect to the grip unit by the first drive unit.

(Item 6) The X-ray imaging apparatus according to item 5, wherein the position holding unit includes an electromagnet and a magnetic body, and holds the grip unit at the predetermined position by a magnetic force between the electromagnet and the magnetic body.

(Item 7) The X-ray imaging apparatus according to any one of items 4 to 6, further including a second moving mechanism that includes a second drive unit and moves the X-ray detection unit in the vertical direction, wherein the control unit is configured to control driving of the second drive unit, and in the adjustment of the position of the X-ray detection unit, after positioning the grip unit, the control unit performs control to relatively move the X-ray detection unit in the vertical direction with respect to the grip unit while holding the position of the grip unit by moving the grip unit and the X-ray detection unit in mutually opposite directions at the same moving speed by the first drive unit and the second drive unit.

(Item 8) The X-ray imaging apparatus according to item 1 or 2, wherein the column includes a guide unit provided so as to extend in the vertical direction, and the support unit moves up and down while being guided by the guide unit together with the first moving mechanism.

(Item 9) The X-ray imaging apparatus according to item 8, further including a connection member connecting the grip unit and the first moving mechanism, wherein the connection member moves up and down while being guided by the guide unit, and the support unit moves up and down while being guided by the guide unit.

(Item 10) An X-ray imaging apparatus including: a column; an X-ray detection unit that detects X-rays transmitted through a subject and is movable in a vertical direction along the column; a support unit that is provided on the column and supports the X-ray detection unit so as to be movable in the vertical direction; a grip unit that is configured to be gripped by a hand of the subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.

### Reference Signs List

1 Subject
2 Predetermined position
10 X-ray irradiation unit
17 Column
18 Guide unit
20 X-ray detection unit
21 Support unit
23 Grip unit
27 Connection member
30 Position holding unit
31 Electromagnet
32 Magnetic body
33 First moving mechanism
34 First drive unit
40 Second moving mechanism
45 Second drive unit
50 Operation unit
51 Control unit
100, 110 X-ray imaging apparatus

## Claims

1. An X-ray imaging apparatus including: an X-ray irradiation unit that irradiates X-rays; a column; an X-ray detection unit that detects the X-rays irradiated from the X-ray irradiation unit and is movable in a vertical direction along the column; a support unit that is provided on the column and supports the X-ray detection unit so as to be movable in the vertical direction; a grip unit that is configured to be gripped by a hand of a subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.

2. The X-ray imaging apparatus according to claim 1, wherein the first moving mechanism is provided on the support unit disposed between the column and the X-ray detection unit.

3. The X-ray imaging apparatus according to claim 1, wherein the first moving mechanism includes a first drive unit, and further includes an operation unit that receives an operation input for relatively moving the grip unit in the vertical direction with respect to the X-ray detection unit by the first drive unit.

4. The X-ray imaging apparatus according to claim 1, wherein the first moving mechanism includes a first drive unit, further includes a control unit that controls driving of the first drive unit, and the control unit adjusts a position of the X-ray detection unit by performing control to move the X-ray detection unit in the vertical direction by the first drive unit while holding a position of the grip unit after the position of the grip unit is positioned at a predetermined position with respect to the column.

5. The X-ray imaging apparatus according to claim 4, further including a position holding unit that holds a vertical position of the grip unit with respect to the column, wherein in the adjustment of the position of the X-ray detection unit, after positioning the grip unit, the control unit performs control to hold the position of the grip unit with respect to the column by the position holding unit and to relatively move the X-ray detection unit in the vertical direction with respect to the grip unit by the first drive unit.

6. The X-ray imaging apparatus according to claim 5, wherein the position holding unit includes an electromagnet and a magnetic body, and holds the grip unit at the predetermined position by a magnetic force between the electromagnet and the magnetic body.

7. The X-ray imaging apparatus according to claim 4, further including a second moving mechanism that includes a second drive unit and moves the X-ray detection unit in the vertical direction, wherein the control unit is configured to control driving of the second drive unit, and in the adjustment of the position of the X-ray detection unit, after positioning the grip unit, the control unit performs control to relatively move the X-ray detection unit in the vertical direction with respect to the grip unit while holding the position of the grip unit by moving the grip unit and the X-ray detection unit in mutually opposite directions at the same moving speed by the first drive unit and the second drive unit.

8. The X-ray imaging apparatus according to claim 1, wherein the column includes a guide unit provided so as to extend in the vertical direction, and the support unit moves up and down while being guided by the guide unit together with the first moving mechanism.

9. The X-ray imaging apparatus according to claim 8, further including a connection member connecting the grip unit and the first moving mechanism, wherein the connection member moves up and down while being guided by the guide unit, and the support unit moves up and down while being guided by the guide unit.

10. An X-ray imaging apparatus including: a column; an X-ray detection unit that detects X-rays transmitted through a subject and is movable in a vertical direction along the column; a support unit that is provided on the column and supports the X-ray detection unit so as to be movable in the vertical direction; a grip unit that is configured to be gripped by a hand of the subject and is provided so as to move in the vertical direction integrally with the X-ray detection unit in accordance with movement of the X-ray detection unit in the vertical direction; and a first moving mechanism that is provided on the support unit and relatively moves the grip unit in the vertical direction with respect to the X-ray detection unit.
